# EUROPÄISCHE PATENTANMELDUNG

(11) **EP 0 555 776 A1**
(43) Veröffentlichungstag der Anmeldung: **18.08.1993**
(21) Anmeldenummer: 93101780.0
(22) Anmeldetag: 05.02.1993
(51) Int. Cl.: C11B 11/00, C07C 59/01, C07C 51/47

(54) **Verfahren zur Isolierung und Reinigung von Fettsäuren und Hydroxyfettsäuren**

(30) Priorität: 13.02.1992 DE 4204321
(71) Anmelder: Beiersdorf Aktiengesellschaft, D-20245 Hamburg (DE)
(72) Erfinder: Paal, Michael, Dr., W-2000 Hamburg 63 (DE); Schmucker, Robert, Dr., W-2000 Hamburg 61 (DE); Rudolph, Martin, Dr., W-2000 Hamburg 62 (DE)

(57) **Zusammenfassung**

Die Erfindung betrifft ein neues Verfahren zur Isolierung und Reinigung von Hydroxyfettsäuren und Fettsäuren ausgehend von Wollwachssäuregemischen.

## Beschreibung

Die Erfindung betrifft ein neues Verfahren zur Isolierung und Reinigung von Hydroxyfettsäuren und Fettsäuren ausgehend von Wollwachssäuregemischen.

Es ist bekannt, daß die aus natürlichem Wollwachs zugänglichen Wollwachssäuren ein komplexes Gemisch darstellen, welches vor allem aus verzweigten und unverzweigten Fettsäuren sowie entsprechenden Hydroxyfettsäuren besteht (K. Motiuk, J. Am. Oil Soc. 56, 91 (1979); G. Barnett, Cosmetics and Toiletries, 101, 23 (1986)).

Zahlreiche Verfahren zur Trennung bzw. Anreicherung dieser Komponenten sind in den letzten Jahrzehnten beschrieben worden. Hierbei handelt es sich oft um destillative Verfahren, die in der Regel gering reproduzierbare Qualitäten liefern und darüber hinaus eine Isolation von thermisch empfindlichen Komponenten nur unbefriedigend gestatten.

Beschrieben sind auch Verfahren, wonach durch Umwandlung der Wollwachssäuren in ihre Kupferchelate Fraktionen von Hydroxyfettsäuren unterschiedlichster Provenienz darstellbar sind oder auch durch Umkristallisieren, fraktionierte Kristallisation, Ausfällung oder fraktionierte Extraktion mit unterschiedlichen Lösungsmittelgemischen versucht wurde, Wollwachssäuregemische in ihre Komponenten aufzutrennen.

Bekannt sind ebenfalls Verfahren, wonach Wollwachssäureester durch Destillation oder durch Flüssigextraktion in hydroxylierte und nicht hydroxylierte Komponenten getrennt werden.

Die für Wollwachssäuren bekannten Verfahren haben den Nachteil, daß sie oft nur als Teilschritte, beispielsweise zur Vorreinigung geeignet sind und sich nachfolgend aufwendige Trennungen z.B. nach Veresterung der Wollwachssäuren anschließen, oder daß die Verfahren insgesamt aufwendig sind und zu lange Zeit erfordern. Nach keinem dieser bekannten Verfahren lassen sich große Mengen von Wollwachssäuregemischen auf wirtschaftliche Weise in die genannten Komponenten zerlegen.

Aufgabe der Erfindung ist es, ein Verfahren zur Isolierung und Reinigung von Hydroxyfettsäuren, insbesondere alpha-Hydroxyfettsäuren und Fettsäuren aus Wollwachssäuregemischen zu schaffen, das die Nachteile des Standes der Technik nicht aufweist. Es soll in wenigen, einfachen Schritten auch von technischen Wollwachssäuregemischen ausgehend, zu den genannten Komponenten führen und ein wirtschaftliches Aufarbeiten auch großer Mengen gestatten.

Dabei sollen die Produkte bzw. Gemische weitgehend rein, kristallin und geruchlos anfallen, so daß ihre direkte Weiterverarbeitung auch in empfindlichen Zubereitungen wie Kosmetika oder Dermatika möglich ist.

Diese Aufgabe wird gelöst durch ein Verfahren zur Trennung und Isolierung von Hydroxyfettsäuren und Fettsäuren aus Wollwachssäuregemischen, das dadurch gekennzeichnet ist, daß man entweder
a) in einem ersten Schritt ein Wollwachssäuregemisch mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht, oder in umgekehrter Reihenfolge
b) in einem ersten Schritt ein Wollwachssäuregemisch mit mindestens einem unpolaren Lösungsmittel behandelt, wobei sich eine Dispersion oder Suspension bildet, den festen Anteil, der die Hydroxyfettsäuren enthält, davon abtrennt und die verbleibende flüssige Phase vom Lösungsmittel befreit, wobei ein Rückstand entsteht der vorwiegend aus nicht hydroxylierten, langkettigen höheren Fettsäuren besteht, und gegebenenfalls in einem zweiten Schritt den verbliebenen festen Anteil mit den Hydroxyfettsäuren mit mindestens einem polaren Lösungsmittel behandelt, wobei sich ein Teil des festen Anteiles löst, die erhaltene Lösung von ungelösten Teilen, die vorwiegend aus nicht hydroxylierten höheren Fettsäuren kurzer Kettenlänge bestehen, befreit und von der Lösung das Lösungsmittel entfernt, wodurch man einen Rückstand erhält, der die Hydroxyfettsäuren enthält, und
c) gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang, vor den Schritten a) oder b), in entsprechender Weise mit dem Wollwachssäuregemisch vornimmt.

Bevorzugt wird die unter a) beschriebene Reihenfolge der Einzelschritte. Das natürliche Wollwachssäuregemisch (oder auch Wollwachssäuren-Gemisch) wird also zuerst mit dem polaren Lösungsmittel und dann mit dem unpolaren Lösungsmittel wie angegeben behandelt.

Auch die in den Verfahrensteilen a) und b) jeweils im ersten Schritt isolierten Gemische enthalten alpha-Hydroxyfettsäuren in höheren Gewichtsanteilen als in den Ausgangsgemischen und können z.B. in kosmetischen Zubereitungen verwendet werden. Vorzugsweise folgen aber die jeweiligen fakultativen genannten zweiten Schritte, die zu einer weiteren Anreicherung der alpha-Hydroxyfettsäuren führen.

Vorzugsweise schließt sich an die Trennungs- und Isolierungsschritte des Verfahrens der fakultative Reinigungsschritt an. Dies kann dann besonders zweckmäßig sein, wenn die erhaltenen Hydroxyfettsäuren und Fettsäuren in kosmetischen Zubereitungen verwendet werden. Auf diese Weise können noch aus dem Wollwachs oder der Wollwachsspaltung herrührende Verunreinigungen entfernt werden. Es ist aber auch möglich, diesen Reinigungsschritt vor Beginn der Isolierungsschritte a) und b) vorzunehmen, also direkt das Wollwachssäuregemisch, das als Ausgangsprodukt dient, einzusetzen.

Das Verfahren bezieht sich vorzugsweise auf alphabeziehungsweise 2-Hydroxyfettsäuren.

Bevorzugte polare Lösungsmittel sind Alkohole und Ketone, vorzugsweise Methanol, Ethanol, n-Propanol, iso-Propanol, Aceton oder Ethylmethylketon, insbesondere Ethanol oder Methanol, sowie Gemische dieser Lösungsmittel, insbesondere mit Methanol oder Ethanol.

Bevorzugte unpolare Lösungsmittel sind aliphatische und cyclische Kohlenwasserstoffe oder alkylierte Aromaten wie Alkylbenzole, vorzugsweise Pentan, Hexan, Heptan und Oktan oder die Isomere dieser Lösungsmittel, Cyclohexan, Toluol oder Xylol, insbesondere aber Heptan oder Cyclohexan, sowie Gemische dieser Lösungsmittel, insbesondere mit Cyclohexan oder Heptan.

Bevorzugt können die erfindungsgemäßen Verfahrensschritte in folgender Weise in an sich bekannter Weise allgemein wie folgt ausgeführt werden:

Das Behandeln eines Säuregemisches oder einer Säurekomponente mit einem Lösungsmittel erfolgt, indem das Lösungsmitel zugegeben und die Stoffe vermischt werden und insbesondere in intensiven, ausreichenden Kontakt gebracht werden, beispielsweise durch Rühren oder Schütteln, vorzugsweise Rühren. Dabei kann sich überwiegend eine Lösung oder eine Suspension oder Dispersion ergeben.

Von diesen Gemischen können feste, ungelöste Anteile abgetrennt werden oder die Lösung kann von ungelösten Anteilen befreit werden. Dies kann z.B. durch Abpressen, Filtrieren oder Zentrifugieren geschehen, insbesondere durch Filtrieren.

Lösungen oder flüssige Phasen können von Lösungsmitteln befreit werden oder das Lösungsmittel kann aus der Lösung entfernt werden. Hierzu wird das Lösungsmittel vorzugsweise unter Erwärmung und/oder vermindertem Druck abgezogen oder abdestilliert.

Das erfindungsgemäße Verfahren wird vorzugsweise wie folgt durchgeführt:
a) Das als Ausgangsprodukt dienende Wollwachssäuregemisch wird mit dem polaren Lösungsmittel vermischt und in intensiven Kontakt gebracht, insbesondere gerührt oder geschüttelt. Hierzu können übliche Rührer und übliche Mischwerke oder Rührwerke verwendet werden. Die Temperatur beträgt dabei vorzugsweise 0°C bis 30°C, insbesondere etwa 20°C.
   Die Lösungsmittel-Gewichtsmenge beträgt vorzugsweise das 1 bis 20-fache, insbesondere 2 bis 10-fache der Wollwachssäuregemisch-Gewichtsmenge. Die Behandlungszeit kann vorzugsweise 0,5 bis 20 Stunden, insbesondere 1 bis 10 Stunden betragen. Man erhält nach Filtration als Rückstand etwa 10 bis 20 Gewichtsprozent des Ausgangsgewichtes nicht hydroxylierte, langkettige höhere Fettsäuren, die insbesondere etwa 18 bis 32 Kohlenstoffatome besitzen.
   Die verbliebene Lösung wird unter Erwärmung bei vermindertem Druck vom Lösungsmittel befreit und es ergibt sich ein Rückstand von etwa 80 bis 90 Gewichtsprozent des Ausgangsgewichtes. Dieser Rückstand wird dann gegebenenfalls mit dem unpolaren Lösungsmittel vermischt und in intensiven Kontakt gebracht, insbesondere gerührt oder geschüttelt. Hierzu können übliche Rührer und Mischwerke oder Rührwerke verwendet werden. Die Temperatur beträgt dabei vorzugsweise 0 bis 30°C, insbesondere etwa 20°C.
   Die Lösungsmittel-Gewichtsmenge beträgt vorzugsweise das 1 bis 20-fache, insbesondere 2 bis 10-fache der Gewichtsmenge des Rückstandes. Die Behandlungszeit kann vorzugsweise 1 bis 40 Stunden, insbesondere 2 bis 20 Stunden betragen. Die sich bildende Lösung wird vom Rückstand z.B. durch erneute Filtration getrennt und der Rückstand kann zweckmäßigerweise einmal oder zweimal mit dem unpolaren Lösungsmittel gewaschen werden. Die Menge beträgt dann etwa 10 Gewichtsprozent der für die Behandlung gewählten Lösungsmittelmenge. Der Rückstand enthält die Hydroxyfettsäuren in einer Menge von etwa 20 Gewichtsprozent, bezogen auf die Ausgangsmenge Wollwachssäuregemisch.
   Die verbleibende Lösung ergibt nach Entfernen des Lösungsmittels bei vermindertem Druck etwa eine Menge von 60 bis 70 Gewichtsprozent des Ausgangsgewichtes eines wachsartigen Rückstandes, der vorwiegend aus nicht hydroxylierten, kurzkettigen höheren Fettsäuren besteht, die insbesondere etwa 14 bis 16 Kohlenstoffatome besitzen.
b) Geht man gemäß der Erfindung in umgekehrter Reihenfolge vor, wird das als Ausgangsprodukt dienende Wollwachssäuregemisch zunächst mit dem unpolaren Lösungsmittel behandelt. Die Temperatur beträgt dabei vorzugsweise 0 bis 30°C, insbesondere etwa 20°C. Die Lösungsmittel-Gewichtsmenge beträgt vorzugsweise das 1 bis 20-fache, insbesondere 2 bis 10-fache des Ausgangsprodukts. Die Behandlungszeit beträgt vorzugsweise etwa 1 bis 40 Stunden, insbesondere 2 bis 20 Stunden.

Der sich bildende Rückstand wird von der Lösung getrennt und die Lösung von dem Lösungsmittel befreit. Der Rückstand, etwa 30 Gewichtsprozent des Ausgangsproduktes, besteht vorwiegend aus nicht hydroxylierten, langkettigen höheren Fettsäuren die insbesondere etwa eine mittlere Anzahl von 24 Kohlenstoffatomen besitzen.

Der zuvor verbliebene Rückstand, etwa 70 Gewichtsprozent, der die Hydroxyfettsäuren enthält, wird zweckmäßigerweise einmal oder zweimal mit dem unpolaren Lösungsmittel gewaschen. Die Menge beträgt dann etwa 10 Gewichtsprozent der für die Behandlung gewählten Lösungsmittelmenge.

Gegebenenfalls wird der wachsartige Rückstand anschließend vorzugsweise mit einem polaren Lösungsmittel gut vermischt und gerührt. Die Temperatur beträgt dabei vorzugsweise 0 bis 30°C, insbesondere etwa 20°C. Die Lösungsmittel-Gewichtsmenge beträgt vorzugsweise das 0,1 bis 20-fache, insbesondere 0,5 bis 10-fache des Rückstandsgewichtes. Die Behandlungszeit beträgt vorzugsweise 0,5 bis 10 Stunden, insbesondere 1 bis 5 Stunden. Man erhält nach Filtration als Rückstand etwa 60 Gewichtsprozent als Ausgangsmenge einer Mischung, die überwiegend, insbesondere zu mehr als 80 Gewichtsprozent, aus vorwiegend nicht hydroxylierten Fettsäuren mit einer mittleren Anzahl von 14 bis 16 Kohlenstoffatomen besteht. Außerdem können sich in dieser Mischung noch wechselnde Anteile Cholesterin und längerkettige 1,2-Diole befinden.

Die verbliebene Lösung wird vom Lösungsmittel befreit und es verbleibt ein Rückstand von etwa 10 Gewichtsprozent (bezogen auf das Ausgangsgewicht) einer Mischung von Hydroxyfettsäuren.

Zur weiteren Reinigung können die Wollwachssäuren bzw. das Gemisch in an sich bekannter Weise über die Bildung der Salzform gereinigt werden. Dazu werden die Säuren mit einer Base, vorzugsweise Alkalihydroxiden wie NaOH, KOH, in Alkoholen, Wasser, oder Gemischen davon, vorzugsweise aber in Alkoholen wie Methanol oder Ethanol, insbesondere bei pH 12 bis 14, z.B. in alkoholischer KOH-Lösung, in die Salze überführt und gelöst. Feste Verunreinigungen werden abfiltriert.

Anschließend wird mit einer Säure, vorzugsweise mit einer Mineralsäure, vorzugsweise 1/10, bis 1/2-normaler Salzsäure, angesäuert und vorzugsweise so viel Wasser bzw. Säure zugegeben, bis ein pH-Wert von etwa 3 und eine deutliche Phasentrennung vorliegen.

Die sich abscheidende Säure wird abfiltriert oder vorzugsweise mit nicht wassermischbaren Lösungsmitteln wie halogenierten, insbesondere chlorierten Kohlenwasserstoffen, z.B. Methylenchlorid oder aber insbesondere mit Essigsäureethylester extrahiert. Man erhält etwa 10 bis 15 Gewichtsprozent bezogen auf das Ausgangsgewicht eines gereinigten Säuregemisches, insbesondere Hydroxyfettsäuregemisches.

Das erfindungsgemäße Verfahren liefert vorzugsweise und weit überwiegend alpha-Hydroxyfettsäuren. Beta-Hydroxysäuren finden sich abhängig vom Ausgangsprodukt nicht oder nur in sehr geringem Maß. Es treten sowohl geradkettige als auch verzweigte alpha-Hydroxyfettsäuren in wechselnden Anteilen auf. Den Hauptanteil bildet mit vorzugsweise 40 bis 90 Gewichtsprozent alpha-Hydroxyhexadecansäure, bezogen auf den Hydroxyfettsäureanteil.

Daneben finden sich Homologe dieser alpha-Hydroxyfettsäuren mit 12 bis 24 Kohlenstoffatomen, und zwar sowohl normale als auch iso-alpha-Hydroxyfettsäuren in wechselnden Anteilen.

Die aus dem natürlichen Wollwachs stammenden alpha-Hydroxyfettsäuren werden in optisch aktiver Form erhalten. Sie liegen vorzugsweise als D-Enantiomere vor, wie beispielsweise die alpha-Hydroxyhexadecansäure.

Vorzugsweise fällt ein Gemisch von alpha-Hydroxyfettsäuren folgender Zusammensetzung an:

| A. | Gewichtsprozent |
|---|---|
| 1. alpha-Hydroxy-hexadecansäure | 40 - 90 |
| 2. alpha-Hydroxy-16-methylheptadecansäure | 1 - 15 |
| 3. alpha-Hydroxy-tetradecansäure | 1 - 10 |
| 4. alpha-Hydroxy-octadecansäure | 1 - 5 |
| 5. alpha-Hydroxy-dodecansäure | 1 - 2 |
| 6. weitere Homologe von 1. | 1 - 20 |

Besonders bevorzugte Gemische enthalten:

| B. | Gewichtsprozent |
|---|---|
| 1. alpha-Hydroxy-hexadecansäure | 70 - 90 |
| 2. alpha-Hydroxy-16-methylheptadecansäure | 8 - 15 |
| 3. alpha-Hydroxy-tetradecansäure | 2 - 6 |
| 4. alpha-Hydroxy-octadecansäure | 1 - 3 |
| 5. alpha-Hydroxy-dodecansäure | 1 - 2 |
| 6. weitere Homologe von 1. | 1 - 10 |

Das erfindungsgemäße Verfahren liefert vorzugsweise ebenso zwei Klassen nicht hydroxylierter Fettsäuren.

Die Klasse der langkettigen verzweigten und unverzweigten höheren Fettsäuren kann z.B. als unlöslicher Anteil in Schritt 1 (a) des Verfahrens in kristalliner Form isoliert werden.

Es handelt sich vorzugsweise um Fettsäuren mit einer C-Zahl von 18-32, wobei das Verteilungsmaximum bei C-24 liegt.

Die Klasse der kurzkettigen, nicht hydroxylierten höheren Fettsäuren fällt z.B. in Schritt 2 (a) des erfindungsgemäßen Verfahrens in Form löslicher Anteile an, die nach Abdestillation des verwendeten Lösungsmittels in fester oder halbfester Form isoliert werden können. Es handelt sich um verzweigte und unverzweigte Fettsäuren mit einer C-Zahl von 12-18, wobei das Verteilungsmaximum bei C-14 bis C-16 liegt.

Gegenstand der Erfindung sind auch die nach dem erfindungsgemäßen Verfahren erhaltenen oder erhältlichen Wollwachssäuren und Wollwachssäuregemische, insbesondere alpha-Hydroxyfettsäuregemische, vorzugsweise die Gemische A und B.

Die als Ausgangsprodukt verwendeten bekannten Wollwachssäuregemische sind als natürliche Produkte unterschiedlich zusammengesetzt. Zweckmäßigerweise werden sie in gereinigter Form beispielsweise in destillierter Form eingesetzt, nachdem sie in üblicher Weise durch Verseifung des Wollwachses erhalten wurden. Besonders bevorzugt können Wollwachssäuregemische aus der Spaltung von Wollfett australischer und neuseeländischer Herkunft verwendet werden.

Die verwendeten Lösungsmittel werden vorzugsweise rein oder technisch rein eingesetzt und sind weitgehend wasserfrei. Es können einzelne Lösungsmittel oder auch Gemische, vorzugsweise von 2 bis 5 Lösungsmitteln, insbesondere 2 oder 3 Lösungsmitteln verwendet werden.

Die Ausgangsprodukte und überraschenderweise auch die in den Zwischenstufen des Verfahrens erhaltenen Säuregemische liegen zumeist wachsartig bis kristallin vor, und sie lassen sich unter üblichen Verfahrensbedingungen und Einsatz üblicher Mechanik wirkungsvoll bearbeiten, insbesondere bei Raumtemperatur.

Das erfindungsgemäße Verfahren liefert überraschend mit wenigen einfachen Schritten weitgehend reine Komponenten der Wollwachssäuregemische und dies auf wirtschaftliche Weise. Sie können z.B. direkt in Kosmetika weiterverarbeitet werden oder als Ausgangsverbindungen zur Herstellung wertvoller Derivate dienen.

Alle Prozentangaben oder Teile beziehen sich, soweit nicht anders angegeben, auf das Gewicht, insbesondere auf das Gesamtgewicht der Zubereitungen.

Gegenstand der Erfindung sind auch die in den Ansprüchen genannten Ausführungsformen.

Die folgenden Beispiele dienen dazu, die Erfindungen zu beschreiben, ohne daß beabsichtigt ist, die Erfindungen auf diese Beispiele zu beschränken.

### Beispiel 1

1 kg destillierte Wollwachssäure wird bei Raumtemperatur in 8 l Methanol zwei Stunden gerührt. Der nach Filtration anfallende Rückstand (200 g) enthält langkettige, vorwiegend nicht hydroxylierte Fettsäuren mit einer C-Zahl von 18 - 32.

Die bei der Filtration anfallende Lösung wird eingeengt und ergibt einen Rückstand von 800 g, der mit 5 l Cyclohexan versetzt und bei Raumtemperatur 10 Stunden gerührt wird. Die sich dabei bildende Lösung wird vom Rückstand durch erneute Filtration getrennt. Dieser Rückstand (a) wird zweimal mit je 1 l Cyclohexan gewaschen und danach getrocknet.

Die Lösung wird eingeengt und ergibt 600 g eines wachsartigen Rückstandes, der vorwiegend aus nicht hydroxylierten, kurzkettigen höheren Fettsäuren mit einer mittleren C-Zahl von 14 - 16 besteht.

Der Rückstand (a) (200 g) wird in 0,7 Liter 10%iger methanolischer Kaliumhydroxidlösung bei 40°C gelöst. Man filtriert und säuert mit halbkonzentrierter wässriger Salzsäure auf pH3 an.

Die Extraktion mit Essigsäureethylester (dreimal mit jeweils 200 ml), Trocknen der organischen Lösung mit Natriumsulfat und Einengen ergeben 140 g eines Rückstandes, der zu ca. (Gewichtsprozent):
1. 83% aus 2-Hydroxyhexadecansäure,
2. 6% aus 2-Hydroxy-16-methylheptadecansäure,
3. 2% aus 2-Hydroxyoctadecansäure,
4. 2% aus 2-Hydroxytetradecansäure,
5. 1% aus 2-Hydroxypentadecansäure,
6. 1% aus 2-Hydroxyheptadecansäure und
7. 5% aus weiteren Homologen von 1 besteht.

### Beispiel 2

1 kg destillierte Wollwachssäure wird bei Raumtemperatur in 6 l Ethanol 1,5 Stunden gerührt. Der nach Filtration anfallende Rückstand (100 g) enthält langkettige vorwiegend nicht hydroxylierte Fettsäuren mit einer C-Zahl von 18 - 32.

Die bei der Filtration anfallende Lösung wird eingeengt und ergibt 900 g Rückstand, der mit 6 l Heptan versetzt und bei Raumtemperatur 14 Stunden gerührt wird. Die sich dabei bildende Lösung wird vom Rückstand durch erneute Filtration getrennt. Dieser Rückstand (a) wird zweimal mit je 2 l Heptan gewaschen und danach getrocknet.

Die Lösung wird eingeengt und ergibt 680 g eines wachsartigen Rückstandes, der vorwiegend aus nicht hydroxylierten kurzkettigen Fettsäuren mit einer mittleren C-Zahl von 14 - 16 besteht.

Der Rückstand (a) (220 g) wird in 0,7 Liter 10%iger methanolischer Kaliumhydroxidlösung bei 40°C gelöst. Man filtriert und säuert mit halbkonzentrierter wäßriger Salzsäure auf pH 3 an.

Die Extraktion mit Essigsäureethylester (dreimal mit jeweils 200 ml), Trocknen der organischen Lösung mit Natriumsulfat und Einengen ergeben 130 g eines Rückstandes der zu ca. (Gewichtsprozent):
1. 78% aus 2-Hydroxyhexadecansäure,
2. 8% aus 2-Hydroxy-16-methylheptadecansäure,
3. 3% aus 2-Hydroxyoctadecansäure,
4. 2% aus 2-Hydroxytetradecansäure,
5. 1% aus 2-Hydroxypentadecansäure,
6. 1% aus 2-Hydroxyheptadecansäure und
7. 7% aus weiteren Homologen von 1 besteht.

### Beispiel 3

1 kg destillierte Wollwachssäure wird bei Raumtemperatur in 7 l Cyclohexan 12 Stunden gerührt. Der sich dabei bildende Rückstand (a) wird von der Lösung durch Filtration getrennt. Die Lösung wird im Vakuum eingeengt. Man erhält 300 g eines weiteren Rückstandes, der aus langkettigen, vorwiegend nicht hydroxylierten Fettsäuren mit einer mittleren C-Zahl von 24 besteht.

Der Rückstand (a) wird mit 0,5 l Cyclohexan gewaschen. Nach Trocknung erhält man 700 g eines wachsartigen Produktes, welches in 1 l Methanol eine Stunde bei Raumtemperatur gerührt wird. Die hiervon resultierende Lösung wird vom Rückstand (b) durch Filtration getrennt und eingeengt. Man erhält dadurch 105 g einer aus 2-Hydroxyfettsäuren bestehenden Mischung folgender Zusammensetzung (Gewichtsprozent):
1. ca. 75% 2-Hydroxy-hexadecansäure,
2. ca. 5% 2-Hydroxy-16-methylheptadecansäure,
3. ca. 1% 2-Hydroxy-octadecansäure,
4. ca. 1% 2-Hydroxy-tetradecansäure,
5. ca. 8% weitere Homologe von 1. und
6. ca. 10% kurzkettige nicht hydroxylierte Fettsäuren.

Der erhaltene Rückstand (b) ergibt 595 g einer Mischung, die zu mehr als 80% aus vorwiegend nicht hydroxylierten Fettsäuren mit einer mittleren C-Zahl von 14 - 16 besteht. Außerdem finden sich in dieser Fraktion wechselnde Anteile Cholesterin und längerkettige 1,2-Diole.

### Beispiel 4

1 kg destillierte Wollwachssäure wird bei Raumtemperatur in 9 l Heptan 12 Stunden gerührt. Der sich dabei bildende Rückstand (a) wird von der Lösung durch Filtration getrennt. Die Lösung wird im Vakuum eingeengt. Man erhält 320 g eines Rückstandes, der aus langkettigen, vorwiegend nicht hydroxylierten Fettsäuren mit einer mittleren C-Zahl von 24 besteht.

Der Rückstand (a) wird mit Heptan gewaschen. Nach Trocknung erhält man 680 g eines wachsartigen Produktes, welches in 1,5 l Ethanol eine Stunde bei Raumtemperatur gerührt wird. Die resultierende Lösung wird vom Rückstand (b) durch Filtration getrennt und eingeengt. Man erhält dadurch 115 g einer aus 2-Hydroxyfettsäuren bestehenden Mischung folgender Zusammensetzung(Gewichtsprozent):
1. ca. 78% 2-Hydroxy-hexadecansäure,
2. ca. 6% 2-Hydroxy-16-methylheptadecansäure,
3. ca. 2% 2-Hydroxy-octadecansäure,
4. ca. 1% 2-Hydroxy-tetradecansäure,
5. ca. 5% weitere Homologe von 1. und
6. ca. 8% kurzkettige nicht hydroxylierte Fettsäuren

Der erhaltene Rückstand (b) ergibt 565 g einer Mischung, die zu mehr als 80% aus vorwiegend nicht hydroxylierten Fettsäuren mit einer mittleren C-Zahl von 14 - 16 besteht. Außerdem finden sich in dieser Fraktion wechselnde Anteile Cholesterin und längerkettige 1,2-Diole.

## Patentansprüche

1. Verfahren zur Trennung und Isolierung von Hydroxyfettsäuren und Fettsäuren aus Wollwachssäuregemischen, dadurch gekennzeichnet, daß man entweder
a) in einem ersten Schritt ein Wollwachssäuregemisch mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht, oder in umgekehrter Reihenfolge
b) in einem ersten Schritt ein Wollwachssäuregemisch mit mindestens einem unpolaren Lösungsmittel behandelt, wobei sich eine Dispersion oder Suspension bildet, den festen Anteil, der die Hydroxyfettsäuren enthält, davon abtrennt und die verbleibende flüssige Phase vom Lösungsmittel befreit, wobei ein Rückstand entsteht der vorwiegend aus nicht hydroxylierten, langkettigen höheren Fettsäuren besteht, und gegebenenfalls in einem zweiten Schritt den verbliebenen festen Anteil mit den Hydroxyfettsäuren mit mindestens einem polaren Lösungsmittel behandelt, wobei sich ein Teil des festen Anteiles löst, die erhaltene Lösung von ungelösten Teilen, die vorwiegend aus nicht hydroxylierten höheren Fettsäuren kurzer Kettenlänge bestehen, befreit und von der Lösung das Lösungsmittel entfernt, wodurch man einen Rückstand erhält, der die Hydroxyfettsäuren enthält, und
c) gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang, vor den Schritten a) oder b), in entsprechender Weise mit dem Wollwachssäuregemisch vornimmt.

2. Verfahren gemäß Anspruch 1, dadurch gekennzeichnet, daß man in einem ersten Schritt ein Wollwachssäuregemisch mit mindestens einem polaren Lösungsmittel behandelt, wobei sich ein Teil des Gemisches löst, die erhaltene Lösung von ungelösten Anteilen abtrennt, wobei die ungelösten Anteile aus vorwiegend nicht hydroxylierten, langkettigen höheren Fettsäuren bestehen und daß man dann das Lösungsmittel aus der verbleibenden Lösung entfernt und einen weiteren Rückstand erhält, der die Hydroxyfettsäuren enthält, und gegebenenfalls in einem zweiten Schritt diesen erhaltenen Rückstand mit mindestens einem unpolaren Lösungsmittel behandelt, wobei sich eine Dispersion oder Suspension bildet, deren festen Anteil, der die Hydroxyfettsäuren enthält, davon abtrennt und die verbleibende flüssige Phase vom Lösungsmittel befreit, wodurch man einen Rückstand erhält, der vorwiegend aus nicht hydroxylierten, kurzkettigen höheren Fettsäuren besteht und gegebenenfalls zur Reinigung jeweils die zuvor erhaltenen Hydroxyfettsäuren oder Fettsäuren in wäßriger Lösung mit einer Base in eine Lösung ihrer Salze überführt, die Lösung von festen Anteilen befreit, dann nach Säurezugabe zur Lösung die Hydroxyfettsäure oder Fettsäure wiedergewinnt und isoliert, oder diese Reinigung gegebenenfalls auch am Anfang in entsprechender Weise mit dem Wollwachssäuregemisch vornimmt.

3. Verfahren gemäß Anspruch 1, dadurch gekennzeichnet, daß als polare Lösungsmittel Alkohole oder Ketone oder Gemische davon verwendet werden.

4. Verfahren gemäß Anspruch 1, dadurch gekennzeichnet, daß als unpolare Lösungsmittel aliphatische oder cyclische Kohlenwasserstoffe oder alkylierte Aromaten oder Gemische davon verwendet werden.

5. Verfahren gemäß Anspruch 1, dadurch gekennzeichnet, daß als polare Lösungsmittel Methanol, Ethanol, n-Propanol, iso-Propanol, Aceton oder Ethylmethylketon und als unpolare Lösungsmittel Pentan, Hexan, Heptan und Oktan oder die Isomeren dieser Lösungsmittel, Cyclohexan, Toluol oder Xylol verwendet werden.

6. Alpha-Hydroxyfettsäuregemische erhältlich nach dem Verfahren gemäß Anspruch 1.

7. Alpha-Hydroxyfettsäuregemische gemäß folgenden Zusammensetzungen A oder B erhältlich nach dem Verfahren gemäß Anspruch 1:
| A. | Gewichtsprozent |
|---|---|
| 1. alpha-Hydroxy-hexadecansäure | 40 - 90 |
| 2. alpha-Hydroxy-16-methylheptadecansäure | 1 - 15 |
| 3. alpha-Hydroxy-tetradecansäure | 1 - 10 |
| 4. alpha-Hydroxy-octadecansäure | 1 - 5 |
| 5. alpha-Hydroxy-dodecansäure | 1 - 2 |
| 6. weitere Homologe von 1. | 1 - 20 |
| B. | Gewichtsprozent |
|---|---|
| 1. alpha-Hydroxy-hexadecansäure | 70 - 90 |
| 2. alpha-Hydroxy-16-methylheptadecansäure | 8 - 15 |
| 3. alpha-Hydroxy-tetradecansäure | 2 - 6 |
| 4. alpha-Hydroxy-octadecansäure | 1 - 3 |
| 5. alpha-Hydroxy-dodecansäure | 1 - 2 |
| 6. weitere Homologe von 1. | 1 - 10 |
